# EUROPÄISCHE PATENTSCHRIFT

(11) **EP 1 710 228 B1**
(45) Veröffentlichungstag und Bekanntmachung des Hinweises auf die Patenterteilung: **08.06.2011**
(21) Anmeldenummer: 06007037.2
(22) Anmeldetag: 03.04.2006
(51) Int. Cl.: C07C 275/62, C07D 229/00, C08G 18/28, C08G 18/32, C08G 18/50, C08G 18/79, C09D 5/04, C09D 7/12, C09D 7/02

(54) **Biuretverbindungen, ihre Herstellung und Verwendung sowie Zwischenprodukte zu ihrer Herstellung**
Biuret compounds, their preparation and use and intermediates for their preparation
Composés biuret, procédé de préparation et utilisation et intermédiaires de préparation

(30) Priorität: 07.04.2005 DE 102005015966
(43) Veröffentlichungstag der Anmeldung: 11.10.2006
(73) Patentinhaber: Byk-Chemie GmbH, 46483 Wesel (DE)
(72) Erfinder: Haubennestel, Karlheinz, 46487 Wesel (DE); Mössmer, Stefan, 46487 Wesel (DE); Orth, Ulrich, 46485 Wesel (DE); Leutfeld, Daniela, 46485 Wesel (DE)
(74) Vertreter: Leifert & Steffan

(56) Entgegenhaltungen:
- EP-A- 1 048 681
- EP-A- 1 593 700
- SINGH, PRITAM ET AL: "Studies on the stability of the dimer of 2,4-tolylene diisocyanate" CANADIAN JOURNAL OF CHEMISTRY, 40, 935-940 CODEN: CJCHAG; ISSN: 0008-4042, 1962, XP008085379
- KRICHELDORF, HANS R. ET AL: "Nitrogen-15 NMR spectroscopy. 27. Spectroscopic characterization of polyurethanes and related compounds" MAKROMOLEKULARE CHEMIE , 182(4), 1177-1196 CODEN: MACEAK; ISSN: 0025-116X, 1981, XP002457060
- REEMERS, SANDRA ET AL: "Novel route to dendritic structures and their application for surface modification" JOURNAL OF POLYMER SCIENCE, PART A: POLYMER CHEMISTRY , 44(4), 1372-1386 CODEN: JPACEC; ISSN: 0887-624X, 2006, XP008085402

## Beschreibung

Die vorliegende Erfindung betrifft Biuretverbindungen, Verfahren zu ihrer Herstellung und ihre Verwendung.

Die erfindungsgemäßen Biuretverbindungen sind als Thixotropierungsmittel für Beschichtungssysteme wie z. B. lösemittelhaltige, lösemittelfreie und wässrige Lacke, PVC-Plastisole, Beschichtungen auf Epoxidbasis und Beschichtungen auf Basis ungesättigter Polyesterharze geeignet.

Um die Rheologie von flüssigen Beschichtungssystemen zu steuern, werden vielfach Kieselsäuren, hydriertes Rizinusöl oder organisch modifizierte Bentonite, wie z.B. in den US-Patenten 4 208 218, 4 410 364 und 4 412 018 beschrieben, eingesetzt. Desweiteren kommen vielfach Polyamidwachse zum Einsatz. Gerade auf dem Gebiet der Polyamide und Polyamidester existieren zahlreiche Patente, wie z.B. DE 69523221, EP 0528363, EP 0239419, US 5,510,452 und US 5,349,011. Es werden aber auch Kombinationen von modifizierten Bentoniten mit Polyamiden, wie in der EP 0509202 und DE 69704691 beschrieben, eingesetzt.

Nachteilig bei diesen Stoffen ist, dass sie meist trockene Feststoffe oder Pasten darstellen, die mittels Lösungsmitteln und unter Verwendung von Scherkräften zu einem Halbfabrikat aufgeschlossen bzw. durch gezielte Temperatursteuerung in das flüssige Beschichtungssystem eingebracht werden müssen. Werden diese Temperaturen nicht eingehalten, treten im fertigen Beschichtungssystem Kristallite auf, die zu Fehlern in der Beschichtung führen. Der generelle Nachteil dieser Systeme ist, daß sie zu Trübungen und Schleierbildungen (Haze) in klaren, transparenten Beschichtungen führen. Außerdem ist der Umgang mit trockenen Produkten, die Stäube bei der Verarbeitung verursachen, nicht gewünscht. Die Polyamidester sind zwar häufig flüssig und daher deutlich weniger wirksam als die von Natur aus festen Stoffe.

Andere Lösungen zur Rheologiesteuerung wurden dargestellt in der Patentanmeldung EP 0 198 519. Hier wird ein Isocyanat mit einem Amin in Gegenwart von Bindemitteln zu einem Harnstoff umgesetzt, der in feinst disperser

Form nadelförmige Kristalle bildet. Diese so modifizierten Bindemittel werden als rheologiesteuernde und ablaufverhindernde Bindemittel angeboten, als sogenannte "sag control agents".

Der Nachteil dieser Produkte liegt darin begründet, dass sie immer an diese Bindemittel gebunden sind, in denen sie hergestellt wurden und nicht eine nachträgliche universelle Korrektur von fertigen Beschichtungsmitteln zulassen.

In der Patentanmeldung EP 0 006 252 wird ein Verfahren zur Herstellung eines Thixotropiemittels beschrieben, das einige der o. g. Nachteile ausräumt, in dem es Harnstoffurethane beschreibt, die in aprotischen Lösungsmitteln in Gegenwart von LiCl durch Umsetzung von Isocyanataddukten mit Polyaminen hergestellt werden. Der Nachteil der so hergestellten Produkte liegt in der durch das Herstellverfahren bedingten undefinierten Struktur dieser Harnstoffurethane. Bei diesem Verfahren wird 1 Mol eines Diisocyanates zunächst mit 1 Mol eines Monoalkohols umgesetzt. Dabei entstehen die gewünschten NCO-funktionellen Monoaddukte, aber auch nicht-NCO-funktionelle Diaddukte. Außerdem bleibt ein gewisser Anteil an monomerem Diisocyanat nicht umgesetzt. Die Anteile dieser verschiedenen Verbindungen können schwanken, je nach Zugänglichkeit der NCO-Gruppe und der angewandten Reaktionsführung, wie Temperatur und Zeit. Alle diese so hergestellten Addukte enthalten jedoch größere Mengen nicht umgesetztes Diisocyanat, welches bei der weiteren Umsetzung mit Polyaminen zu unkontrollierter Kettenverlängerung des Moleküls führt. Diese Produkte neigen dann zu Ausfällungserscheinungen oder vorzeitiger Gelierung und demzufolge zur Bildung von sogenannten "Stippen" ("seeds") im Bindemittel. In der Patentanmeldung DE 19919482 werden diese Nachteile durch Entfernen des überschüssigen Isocyanates umgangen. Diese Produkte haben jedoch den Nachteil, dass sie nur in hochpolaren Lösemitteln wie z.B. N-Methylpyrrolidon unter Zuhilfenahme von Alkalisalzen stabile Lösungen liefern.

EP 1048681 beschreibt als Thixotropie-Mittel wirksame, Harnstoffurethane enthaltende Lösungen.

Die vorliegenden Erfindung hat sich daher die Aufgabe gestellt, ein Verfahren zu finden, welches Thixotropiemittel einer definierten Struktur erzeugt und damit ein besseres Wirkungsprofil und eine bessere Reproduzierbarkeit der Thixotropierung gewährleistet.

Überraschenderweise wurde gefunden, dass diese Aufgabenstellung gelöst werden kann, durch Biuretverbindungen, die aus Uretdionen (Komponente A) und Diaminen (Komponente B) herstellbar sind und die allgemeine Struktur A-B-A aufweisen.

Gegenstand der Erfindung sind daher Biuretverbindungen der allgemeinen Formel 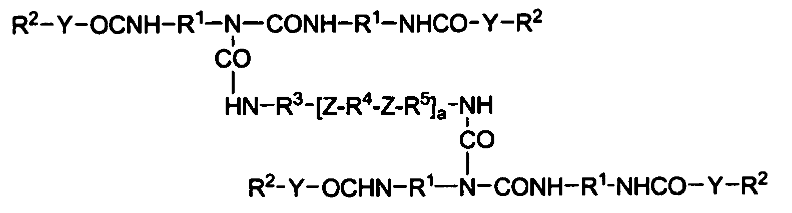 in der bedeuten:
R¹ = C₂-C₁₈-Alkylen, Cycloalkylen, Arylen oder Aralkylen,
Y = -O- und/oder -NH-,
R² = C₄-C₂₂-Alkyl, C₃-C₁₈-Alkenyl, Cycloalkyl, Aralkyl, CₘH₂ₘ₊₁(O-CₙH₂ₙ)ₓ -(O-CH(C₆H₅)-CH₂)ᵤ-, CₘH₂ₘ₊₁(OOC-CᵥH₂ᵥ)ₓ-, X-C₆H₄-(O-CₙH₂ₙ)x-(O-CH(C₆H₅)-CH₂)ᵤ₋, wobei m = 1-22, n = 2-4, x = 0-15, u = 0-15, v = 4-5 ist,
X = C₁-C₁₂-Alkyl, -(C₆H₅)₁₋₄ und
R³, R⁴ und R⁵ = C₂-C₁₈-Alkylen, Cycloalkylen, Arylen oder Aralkylen, wobei R³, R⁴ und R⁵ gleich oder verschieden sein können,
Z = -COO-, NHCO-, NHCOO-, NHCONH- und/oder Mischungen davon
und wobei a = 1-20 ist.

Vorteilhaft ist der Rest R² ein Polyalkoxymonoalkoholrest, der Ethylenoxid- und/oder Propylenoxid- und/oder Butylenoxidgruppen enthält.

Die Erfindung betrifft auch ein Verfahren zur Herstellung der Biuretverbindungen, bei dem Uretdione der allgemeinen Formel (A) 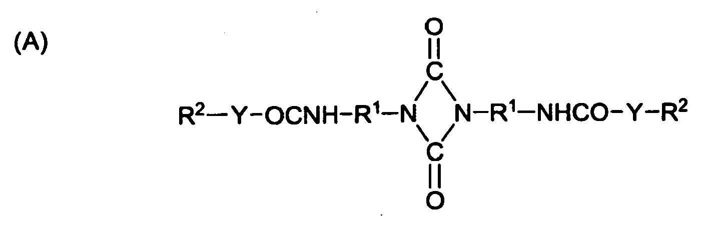 in der bedeuten:
R¹ = C₂-C₁₈-Alkylen, Cycloalkylen, Arylen oder Aralkylen,
Y = -O- und/oder -NH-,
R² = C₄-C₂₂-Alkyl, C₃-C₁₈-Alkenyl, Cycloalkyl, Aralkyl, CₘH₂ₘ₊₁(O-CₙH₂ₙ)ₓ -(O-CH(C₆H₅)-CH₂)ᵤ-, CₘH₂ₘ₊₁(OOC-CᵥH₂ᵥ)ₓ-, X-C₆H₄-(O-CₙH₂ₙ)x-(O-CH(C₆H₅)-CH₂)ᵤ-,
wobei m = 1-22, n = 2-4, x = 0-15, u = 0-15, v = 4-5 ist,
X = Alkyl-(C₁-C₁₂), -(C₆H₅)₁₋₄,
mit Diaminen der allgemeinen Formel B

(B) H₂ N-R³-[Z-R⁴-Z-R⁵]ₐ-NH₂

in der bedeuten:
R³, R⁴ und R⁵ = C₂-C₁₈-Alkylen, Cycloalkylen, Arylen oder Aralkylen,
wobei R³, R⁴ und R⁵ gleich oder verschieden sein können,
Z = -COO-, NHCO-, NHCOO-, NHCONH- und/oder Mischungen davon
und wobei a = 1-20 ist,
umgesetzt werden.

Bei der Herstellung der erfindungsgemäßen Biuretverbindungen werden uretdionhaltige Polyisocyanate zunächst unter Erhalt der Uretdiongruppierung mit Monoalkoholen und/oder Monoaminen zu urethan- und/oder harnstoffhaltigen Polymeren umgesetzt, und in einem zweiten Schritt werden durch Umsetzung mit Polyaminen unter Ringöffnung des Uretdion-Ringes die erfindungsgemäßen Biuretverbindungen hergestellt.

Uretdione werden, wie dem Fachmann bekannt, durch Addition von monomeren Diisocyanaten unter Verwendung bestimmter Katalysatoren hergestellt (Laas, H.J.; Halpaap, R.; Pedain,J.; J. prakt. Chemie 336 (1994), 185-200). Bevorzugt ist die Verwendung von HDI-Uretdion, welches kommerziell als Desmodur^{®} N 3400 von BAYER verfügbar ist und neben der Uretdiongruppierung zusätzlich HDI-Trimerisate und Allophanate aufweist.

Bei den im ersten Schritt eingesetzten Monoalkoholen handelt es sich um aliphatische, cycloaliphatische und araliphatische Alkohole. Bei den aliphatischen Alkoholen werden lineare, verzweigte oder cyclische Alkohole der Kettenlänge C2-C22 eingesetzt, wie z. B Ethanol, Propanol, n-Butanol, Oktanol, Decanol, Dodecanol, Oleylalkohol und Stearylalkohol. Cycloaliphatische Alkohole umfassen z.B. Cyclopentanol und Cyclohexanol. Araliphatische Alkohole wie z.B. Benzylalkohol finden ebenfalls Verwendung. Polymere Alkohole wie Polyolefinmonoole, Polyacrylatmonoole, Polycarbonatmonoole, Polycaprolactonmonoole oder Polysiloxanmonoole können ebenso bei der Erfindung verwendet werden wie Fettalkoholalkoxylate mit variablem Alkoxylierungsgrad, wie sie dem Fachmann unter dem Handelsnamen Lutensol^{®} der BASF bekannt sind. Bevorzugt werden Polyalkoxymonoalkohole verwendet, welche Ethylenoxid- und/oder Propylenoxid- und/oder Butylenoxidgruppen enthalten und gegebenenfalls mit Styroloxid modifiziert sind. Besonders bevorzugt ist die Verwendung von Polyalkoxymonoalkoholen wie z.B: MPEG 350, MPEG 500 und MPEG 750, bei denen es sich um Methanol gestartete Polyethylenglykole mit einer terminalen OH-Gruppe handelt. Die Monoalkohole können auch in Mischungen eingesetzt werden.

Bei den Monoaminen handelt es sich um aliphatische, cycloaliphatische und araliphatische Amine. Bei den aliphatischen Aminen werden lineare, verzweigte oder cyclische Amine der Kettenlänge C2 - C22 eingesetzt, wie z. B. Ethylamin, Propylamin, Isopropylamin, Butylamin, sek. und tert.-Butylamin, 3-Methyl-1-butanamin, Hexylamin, 2-Ethylhexylamin, Octylamin, Cyclopentylamin, Cyclohexylamin, Tridecylamin, Oleylamin, Octadecylamin und die unter dem Handelsnamen Armeen^{®} von Akzo Nobel bekannten Mischungen von C12 - C 22 Aminen. Erfindungsgemäß sind neben Polyolefinaminen wie z.B. Polyisobutylenamin auch bevorzugt Polyoxyalkylenmonoamine, welche Ethylenoxid- und/oder Propylenoxidgruppen enthalten und welche unter den Handelsnamen Jeffamine^{®} M 600, M 1000, M 2005 und M 2070 von Huntsman bekannt sind. Bei den araliphatischen Aminen handelt es sich um Produkte wie z. B. Benzylamin und Furfurylamin. Es können aber auch Hydrazide wie z.B. Benzoesäurehydrazid eingesetzt werden. Die Monoamine können auch als Mischungen eingesetzt werden, ebenso können die Monoamine mit den Monoalkoholen in jedem Verhältnis gemischt, eingesetzt werden.

Die Reaktion zwischen dem uretdionhaltigen Polyisocyanat und dem Monoalkohol, unter Erhalt des Uretdionringes, wird bei Temperaturen zwischen 15 und 60°C, bevorzugt zwischen 20 und 50°C, gegebenenfalls unter Zuhilfenahme eines Katalysators, wie Dibutylzinndilaurat (DBTL), durchgeführt. Die Reaktion zwischen dem uretdionhaltigen Polyisocyanat und dem Monoamin unter Erhalt des Uretdionringes wird bei Temperaturen zwischen 15 und 45°C, bevorzugt zwischen 20 und 30 °C durchgeführt. Dabei ist die Reihenfolge der Zugabe der Co-Reaktanden im allgemeinen beliebig. Das uretdionhaltige Polyisocyanat kann vorgelegt werden, gegebenenfalls in einem inerten Lösemittel, und der Monoalkohol oder das Monoamin werden zugetropft. Ebenso kann der Monoalkohol oder das Monoamin vorgelegt werden, und das uretdionhaltige Polyisocyanat wird zugetropft. Wird dagegen eine Mischung von Monoalkohol und Monoamin eingesetzt, wird das uretdionhaltige Polyisocyanat zuerst mit dem Alkohol und danach mit dem Amin umgesetzt. Gegebenenfalls kann die Reaktion auch in einem inerten Lösemittel durchgeführt werden, wie z.B. Methyoxypropylacetat, Cyclohexan, Toluol, Xylol oder einem höhersiedenden Aromaten wie z.B. Shellsol^{®} A. N-Methylpyrrolidon oder N-Ethylpyrrolidon sind ebenfalls als Lösemittel geeignet.

Die Diamine der allgemeinen Formel (B) werden dadurch hergestellt, dass man Polycarbonsäuren, bevorzugt Dicarbonsäuren, und/oder Dicarbonsäureanhydride mit Diaminen umsetzt, wobei das Verhältnis Diamin:Polycarbonsäure zwischen 2:1 und 20:9, bevorzugt zwischen 3:2 und 12:11 und besonders bevorzugt zwischen 4:3 und 8:7 liegt.

Bei den Diaminen handelt es sich vorzugsweise um aliphatische und araliphatische primäre Diamine, wie z. B. Ethylendiamin, Neopentandiamin, 1,2- und 1,3-Propandiamin, 1,6-Hexamethylendiamin, 1,8-Octamethylendiamin, 1,12-Dodecamethylendiamin, Cyclohexyldiamin, 4,4'-Diaminodicyclohexylmethan, 3,3'-Dimethyl-4,4'-Diaminodicyclohexylmethan, Isophorondiamin, 4,7-Dioxadecan-1,10-diamin, 4,7,10-Trioxadecan-1,13-diamin, Polyoxyalkylendiamine, welche Ethylenoxid- und/oder Propylenoxid-Gruppen, statistisch oder blockweise angeordnet, enthalten, bekannt unter den Markennamen Jeffamine^{®} D und Jeffamine^{®} ED von Huntsman, mit einem zahlenmittleren Molekulargewicht zwischen 148 und 4000 g/mol, para -und meta-Xylylendiamin. Bevorzugt ist 1,6-Hexamethylendiamin. Es können aber auch Hydrazide wie z.B. Oxalsäuredihydrazid, Bernsteinsäuredihydrazid oder Adipinsäuredihydrazid eingesetzt werden. Mischungen dieser Diamine sind ebenfalls möglich.

Bei den Polycarbonsäuren handelt es sich vorzugsweise um aliphatische, cycloaliphatische oder aromatische, lineare oder verzweigte, gesättigte oder ungesättigte Dicarbonsäuren mit wenigstens 2, bevorzugt zwischen 3 und 40 C-Atomen. Beispiele solcher Polycarbonsäuren sind Adipinsäure, Oxalsäure, Malonsäure, Bernsteinsäure, Glutarsäure, Pimelinsäure, Korksäure, Sebacinsäure, Azelainsäure, Undecandisäure, 1,11- Undecandicarbonsäure, Dodecandisäure, Hexadecandisäure, Dokosandisäure Maleinsäure, Fumarsäure, Terephthalsäure oder Isophthalsäure, alleine oder in Mischungen eingesetzt. Säureanhydride wie Maleinsäureanhydrid, Glutarsäureanhydrid, Phthalsäureanhydrid und Bernsteinsäureanhydrid, welche gegebenenfalls mit Alkyl- oder Alkylengruppen modifiziert sind wie z. B. Dodecenylbernsteinsäureanhydrid, sind ebenfalls geeignet. Polymere Polycarbonsäuren wie z.B. die Dicarbonsäure des Polybutadiens können ebenso verwendet werden wie hydroxyfunktionelle Polycarbonsäuren wie z. B. Weinsäure, Zitronensäure und Hydroxyphthalsäure. Oxydicarbonsäuren wie 3,6,9-Trioxyundecandisäure und Polyglykoldisäure sind ebenfalls mit eingeschlossen. Dimerisierte Fettsäuren, dem Fachmann als Dimersäuren bekannt, mit einer Kohlenstofflänge von 36 C-Atomen sind bevorzugt. Diese Dimersäuren können sowohl einen geringen Monomergehalt (üblicherweise < 8 Gewichtsprozent), als auch einen Anteil von nicht mehr als 20 Gewichtsprozent an Trimersäure aufweisen.

Die Polycarbonsäuren können teilweise oder ganz durch Diisocyanate und die Diamine teilweise oder ganz durch Diole ersetzt werden, wobei dann Ester-, Urethan- und/oder Harnstoffgruppen neben den bevorzugten Amidgruppierungen im Polymer vorliegen können.

Bei den Diolen handelt es sich vorzugsweise um Polyalkylenpolyole, Polyalkenylpolyole, gegebenenfalls mit C1-C4 Alkyl- und/oder Alkoxygruppen modifiziert, um Polyetherpolyole, Polyesterpolyole, gemischte Polyesterpolyetherpolyole, Polycarbonatpolyole, Polyolefinpolyole, Polyacrylatpolyole, Polycaprolactonpolyole und Polysiloxanpolyole mit vorzugsweise 2 Hydroxylendgruppen.

Als Diisocyanate können aliphatische, cycloaliphatische und aromatische Diisocyanate, alleine oder in Mischungen eingesetzt werden. Beispiele solcher Diisocyanate sind 1,4-Tetramethylendiisocyanat, 1,6-Hexamethylendiisocyanat, 2,2,4-Trimethyl-1,6-Hexamethylendiisocyanat, 1,10-Decamethylendiisocyanat, 1,4,-Cyclohexylendiisocyanat, p-Phenylen-Diisocyanat, m-Phenylen-Diisocyanat, 2,6-Toluendiisocyanat, 2,4-Toluendiisocyanat und deren Mischungen, p- und m-Xylilendiisocyanat, 4-4',-Diisocyanatodicyclohexylmethan, 3,3'-Dimethyl-4,4'-Bisphenylendiisocyanat, 3,3'-Dimethyl-Diisocyanatodiphenylmethan, die lsomerenmischungen 2,4'-und 4-4'-Diisocyanatodiphenylmethan und C₃₆ Dimerdiisocyanat.

Die Diamine der allgemeinen Formel (B) werden unter Bedingungen hergestellt, wie sie dem Fachmann bekannt sind. Die Reaktionstemperatur bei der Kondensationsreaktion der Dicarbonsäuren mit Diaminen/Diolen liegt bevorzugt zwischen 100 und 250 °C, besonders bevorzugt zwischen 140 und 200 °C. Das Verhältnis von Diamin und Polycarbonsäure wird vorzugsweise so gewählt, dass auf n Äquivalente Polycarbonsäure (n + 1) Äquivalente Diamin verwendet werden, so dass das Kondensationsprodukt nach Beendigung der Reaktion eine Aminzahl aufweist. Das Verhältnis Diamin : Polycarbonsäure liegt zwischen 2:1 und 20:19, bevorzugt zwischen 3:2 und 12:11, besonders bevorzugt zwischen 4:3 und 8:7. Bei der Additionsreaktion von Diisocyanaten mit Diaminen/Diolen liegt die Reaktionstemperatur bevorzugt zwischen 40 und 120°C, besonders bevorzugt zwischen 60 und 100°C.

Zur Herstellung der erfindungsgemäßen Biuretverbindungen werden die Uretdione der allgemeinen Formel (A) und die Diamine der allgemeinen Formel (B) bei einer Reaktionstemperatur zwischen 60 und 120 °C, besonders bevorzugt zwischen 75 und 90°C zur Reaktion gebracht. Dabei wird das Verhältnis der Komponenten A und B so gewählt, dass auf 1 Mol A zwischen 0,3 Mol und 0,7 Mol, bevorzugt zwischen 0,4 Mol und 0,6 Mol, besonders bevorzugt 0,5 Mol B eingesetzt werden. Die Reaktion kann mit oder ohne Lösemittel durchgeführt werden. Als Lösemittel geeignet sind alle aliphatischen, aromatischen, protischen und aprotischen Lösemittel wie z.B. Methyoxypropylacetat, Cyclohexan, Toluol, Xylol oder höhersiedende Aromaten wie z.B. Shellsol^{®} A. N-Methylpyrrolidon oder N-Ethylpyrrolidon, aber auch Alkohole wie Ethanol, Propanol, i-Butanol oder Butylglykol sind ebenfalls geeignet. Auch Mischungen von Lösemitteln lassen sich verwenden.

Gegenstand der Erfindung ist weiterhin die Verwendung der vorstehend beschriebenen Additionsverbindungen als Mittel zur Rheologiesteuerung, insbesondere als Antiablaufmittel und Antiabsetzmittel, insbesondere bei der Verwendung von schweren Pigmenten, die zum harten Absetzen neigen wie Aluminiumpigmente und Micapigmente.

Aber auch bei Kunststoffbeschichtungen für Industriefußböden auf Epoxyd- oder Polyurethanbasis oder bei sogenannten Gel Coats auf Basis ungesättigter Polyesterharze sind rheologiesteuernde Mittel von Vorteil und die erfindungsgemäßen Additionsverbindungen lassen sich hier vorteilhaft einsetzen.

Im folgenden wird die Erfindung anhand von Ausführungsbeispielen näher erläutert.

### Herstellung der Komponente A:

### Beispiel 1:

In einem 1-Liter 3-Halskolben mit Rührer, Rückflußkühler und Thermometer werden bei Raumtemperatur nacheinander 79,6g (0,2 mol) Hexamethylendiisocyanat-Uretdion (Desmodur^{®} N3400 der Fa. Bayer) und 300g (0,4 mol) Methoxypolyethylenglykol 750 eingefüllt und auf 80°C erwärmt. Die Reaktion wird solange geführt bis kein Isocyanat mehr nachweisbar ist. Dann wird auf 50°C abgekühlt.

### Herstellung der Komponente B:

### Beispiel 2:

In einem 1-Liter 3-Halskolben mit Rührer, Wasserabscheider und Thermometer werden nacheinander 168g (0,3 mol) Dimersäure, 46,4g (0,4 mol)Hexamethylendiamin und 92g Shellsol^{®} A (hocharomatische Kohlenwasserstofflösemittel, Shell) eingewogen und langsam auf 160°C erhitzt. Das bei der Reaktion langsam freiwerdenede Wasser wird azeotrop über den Wasserabscheider abgetrennt. Die Reaktion ist beendet, wenn die Säurezahl < 3 ist. Danach wird die Reaktionsmischung auf 50°C abgekühlt.

### Herstellung der Biuret-Addukte:

### Beispiel 3:

In einem 1-Liter 3-Halskolben mit Rührer, Rückflußkühler und Thermometer werden nacheinander 103,6g (0,1 mol) des Reaktionsproduktes aus Beispiel 1 und 153,1 (0,05 mol) des Reaktionsproduktes aus Beispiel 2 eingewogen und auf 80°C erwärmt. Die Reaktionsmischung wird weitere 3 Std. gerührt, bis die Aminzahl < 3 ist. Dann wird das Produkt mit i-Butanol auf 50% Festkörper verdünnt.

### Komponenten A:

| **Beispiel** | **Uretdion** | **Amin- / Alkoholkomponenten** |
|---|---|---|
| 4 | Hexamethylendiisocyanat-Uretdion | Oleylalkohol |
| 5 | Hexamethylendiisocyanat-Uretdion | Oleylamin |
| 6 | Hexamethylendiisocyanat-Uretdion | Jeffamin^{®} M 600 |
| 7 | Hexamethylendiisocyanat-Uretdion | Methoxypolyethylenglykol M350 |
| 8 | Hexamethylendiisocyanat-Uretdion | Methoxypolyethylenglykol M500 |
| 9 | Hexamethylendiisocyanat-Uretdion | Jeffamin^{®} M2070 |
| 10 | | |

### Komponenten B:

| **Beispiel** | **Dicarbonsäure** | **Diamin** | **Molverhältnis Dicarbons. : Diamin** |
|---|---|---|---|
| 11 | Dimersäure | Jeffamin^{®} ED 600 | 4 : 5 |
| 12 | Adipinsäure | Jeffamin^{®} ED 900 | 5 : 6 |
| 13 | Dimersäure | Jeffamin^{®} ED 2003 | 5 : 6 |
| 14 | Adipinsäure | m-Xylylendiamin | 3 : 4 |
| 15 | Adipinsäure | Jeffamin^{®} ED 900 | 4 : 5 |
| 16 | Adipinsäure | Jeffamin^{®} ED 2003 | 3 : 4 |
| 17 | Adipinsäure / Dimersäure Verhältnis 2.1 | Jeffamin^{®} ED 600 | 4 : 5 |
| 18 | Adipinsäure / Dimersäure Verhältnis 2.1 | Jeffamin^{®} ED 2003 | 4 : 5 |
| 19 | Adipinsäure / Dimersäure Verhältnis 2.1 | m-Xylylendiamin | 4 : 5 |
| 20 | Adipinsäure | Hexamethylendiamin | 4:5 |
| 21 | Dimersäure | Hexamethylendiamin | 4:5 |
| 22 | Dimersäure | m-Xylylendiamin | 4 : 5 |
| 23 | Dimersäure | m-Xylylendiamin / Hexamethylendiamin 3:2 | 4 : 5 |

### Biuret-Addukte:

| **Beispiel** | **Komponente A** | **Komponente B** |
|---|---|---|
| I | aus Beispiel 1 | aus Beispiel 2 |
| II | aus Beispiel 6 | aus Beispiel 11 |
| III | aus Beispiel 9 | aus Beispiel 13 |
| IV | aus Beispiel 1 | aus Beispiel 19 |
| V | aus Beispiel 5 | aus Beispiel 12 |

### Anwendungstechnische Ergebnisse:

Die erfindungsgemäßen Produkte wurden in 2-Komponenten - Epoxy - Systemen auf ihre Fähigkeit zur Ausbildung von Gelen überprüft. Desweiteren wurden die erreichbaren Schichtdicken in den Bindemitteln ermittelt. Folgendes 2K - Bindemittelsystem wurde eingesetzt:
   1. Epikote^{®} 1001 / Epikote^{®} 834, Polyepoxide auf der Basis von Bisphenol A,
      Ancamide^{®} 700-X-75, höhermolekulares Diamin
   2. Epicote^{®} 828, Polyepoxide auf der Basis von Bisphenol A,
      Ancamide^{®} 700-X-75, höhermolekulares Diamin

### Testformulierung:

| | |
|---|---|
| Epikote^{®},1001 (75%ig) in Xylol | 34,5 g |
| Epikote^{®} 834 (80%ig) in Xylol | 7,6 g |
| Byk^{®} 052 (Entschäumer) | 0,3 g |
| Bayferrox^{®} 130M (Eisenoxid-Pigment) | 5,0 g |
| Micro Talc^{®} AT-1 (CaCO3) | 12,0 g |
| Zinkphosphat | 12,0 g |
| EWO (BaSO4) | 5,6 g |

### Lösemittelmischung:

| | |
|---|---|
| Methylisobutylketon | 19,2 g |
| i-Butanol | 3,8 g |

### Härter:

### Ancamide^{®} 700X75

Unter starkem Rühren (Dispermat^{®}, 30 Min. / 8500 sec.⁻¹) werden 1% der erfindungsgemäßen Produkte in die Testformulierung eingerührt. Danach wird auf 20°C abgekühlt und mit der Lösemittelmischung verdünnt.
Dann wird der Gel-Aufbau bewertet.

Nach 24 Std. wird der Härter unter Rühren zugegeben und die Formulierung mit einer Stufenrakel aufgetragen. Nach Aushärtung wird die erreichte Schichtdicke beurteilt.

| **Beispiel** | **2-K-System** | **Gelstärke** | **Schichtdicke** |
|---|---|---|---|
| Control | 1 | kein Gel | 200 µm |
| Byk^{®} 410 | 1 | kein Gel | 450 µm |
| Control | 2 | kein Gel | 100 µm |
| Byk^{®} 410 | 2 | kein Gel | 300µm |
| Beispiel I | 1 | starkes Gel | 800 µm |
| Beispiel II | 1 | starkes Gel | 850 µm |
| Beispiel III | 2 | starkes Gel | 750 µm |
| Beispiel IV | 1 | starkes Gel | 1000 µm |
| Beispiel V | 2 | starkes Gel | 800 µm |

### Erläuterungen der Handelsnamen:

| | |
|---|---|
| Epikote^{®} 1001: | Polyepoxid auf Basis Bisphenol A (Shell) |
| Epikote^{®} 834: | Polyepoxid auf Basis Bisphenol A (Shell) |
| Byk^{®} 410: | Harnstoffurethan, Anlösung in N-Methylpyrrolidon |
| Jeffamin^{®} M 600: | Alkylpolyetheramin, MG 600 (Huntsman) |
| Jeffamin^{®} M 2070: | Alkylpolyetheramin, MG 2000 (Huntsman) |
| Jeffamin^{®} ED 900: | Alkylpolyetherdiamin, MG 900 (Huntsman) |
| Jeffamin^{®} ED 2003: | Alkylpolyetherdiamin, MG 2000 (Huntsman) |
| Ancamide^{®} 700-X-75: | Polyamid/Epoxid-Addukt (Air Products) |

## Patentansprüche

1. Biuretverbindungen der allgemeinen Formel 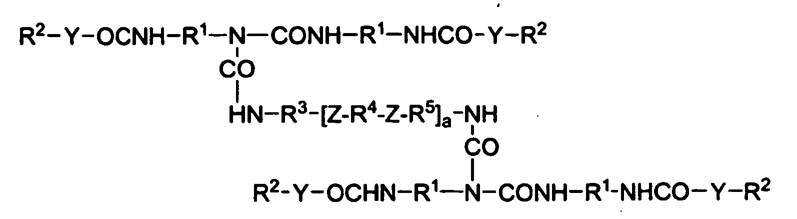 in der bedeuten:
R¹ = C₂-C₁₈-Alkylen, Cycloalkylen, Arylen oder Aralkylen,
Y = -O- und/oder -NH-,
R² = C₄-C₂₂-Alkyl, C₃-C₁₈-Alkenyl, Cycloalkyl, Aralkyl, CₘH₂ₘ₊₁(O-CₙH₂ₙ)ₓ -(O-CH(C₆H₅)-CH₂)ᵤ-, CₘH₂ₘ₊₁(OOC-GᵥH₂ᵥ)ₓ-, X-C₆H₄-(O-CₙH₂ₙ)x-(O-CH(C₆H₅)-CH₂)ᵤ-,
wobei m = 1-22, n = 2-4, x = 0-15, u = 0-15, v = 4-5 ist,
X = C₁-C₁₂-Alkyl, -(C₆H₅)₁₋₄ und
R³, R⁴ und R⁵ = C₂-C₁₈-Alkylen, Cycloalkylen, Arylen oder Aralkylen, wobei R³, R⁴ und R⁵ gleich oder verschieden sein können,
Z = -COO-, NHCO-, NHCOO-, NHCONH- und/oder Mischungen davon und wobei a = 1-20 ist.

2. Biuretverbindungen nach Anspruch 1, **dadurch gekennzeichnet, dass** der Rest R² ein Polyalkoxymonoalkoholrest ist, der Ethylenoxid- und/oder Propylenoxid- und/oder Butylenoxidgruppen enthält.

3. Verfahren zur Herstellung der Biuretverbindungen nach Anspruch 1, **dadurch gekennzeichnet, dass** Uretdione der allgemeinen Formel (A) 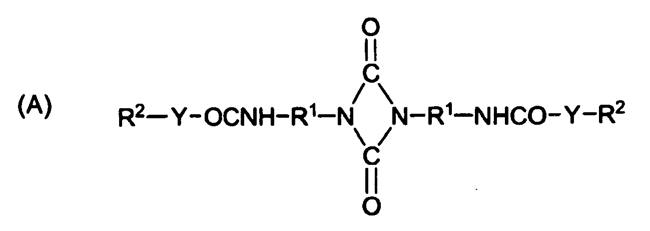 in der bedeuten:
R¹ = C₂-C₁₈-Alkylen, Cycloalkylen, Arylen oder Aralkylen,
Y = -O- und/oder -NH-,
R² = C₄-C₂₂Alkyl, C₃-C₁₈-Alkenyl, Cycloalkyl, Aralkyl, CₘH₂ₘ₊₁(O-CₙH₂ₙ)ₓ -(O-CH(C₆H₅)-CH₂)ᵤ-, CₘH₂ₘ₊₁(OOC-CᵥH₂ᵥ)ₓ-, X-C₆H₄-(O-CₙH₂ₙ)x-(O-CH(C₆H₅)-CH₂)ᵤ-,
wobei m = 1-22, n = 2-4, x = 0-15, u = 0-15, v = 4-5 ist, und
X = C₁-C₁₂-Alkyl oder -(C₆H₅)₁₋₄,
mit Diaminen der allgemeinen Formel B
(B) H₂N-R³-[Z-R⁴-Z-R⁵]ₐ-NH₂
in der bedeuten:
R³, R⁴ und R⁵ = C₂-C₁₈-Alkylen, Cycloalkylen, Arylen oder Aralkylen,
wobei R³, R⁴ und R⁵ gleich oder verschieden sein können,
Z = -COO-, NHCO-, NHCOO-, NHCONH- und/oder Mischungen davon und wobei a = 1-20 ist,
umgesetzt werden.

4. Verfahren zur Herstellung der Biuretverbindungen nach Anspruch 3, **dadurch gekennzeichnet, dass** Verbindungen der Formeln (A) und (B) bei einer Reaktionstemperatur zwischen 60°C und 120°C umgesetzt werden.

5. Verfahren zur Herstellung der Biuretverbindungen nach den Ansprüchen 3 und 4, **dadurch gekennzeichnet, dass** das molare Verhältnis des urethan- und/oder harnstoffhaltigen Polymers zu Polyamin zwischen 1:0,3 und 1:0,7 beträgt.

6. Verwendung von Biuretverbindungen der allgemeinen Formel 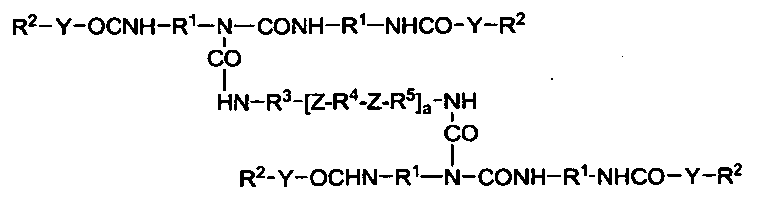 in der bedeuten:
R¹ = C₂-C₁₈-Alkylen, Cycloalkylen, Arylen oder Aralkylen,
Y = -O- und/oder -NH-,
R² = C₄-C₂₂-Alkyl, C₃-C₁₈-Alkenyl, Cycloalkyl, Aralkyl, CₘH₂ₘ₊₁(O-CₙH₂ₙ)ₓ -(O-CH(C₆H₅)-CH₂)ᵤ-, CₘH₂ₘ₊₁(OOC-CᵥH₂ᵥ)ₓ-, X-C₆H₄-(O-CₙH₂ₙ)x-(O-CH(C₆H₅)-CH₂)ᵤ-,
wobei m = 1-22, n = 2-4, x = 0-15, u = 0-15, v = 4-5 ist,
X = C₁-C₁₂-Alkyl, -(C₆H₅)₁₋₄ und
R³, R⁴ und R⁵ = C₂-C₁₈-Alkylen, Cycloalkylen, Arylen oder Aralkylen,
wobei R³, R⁴ und R⁵ gleich oder verschieden sein können,
Z = -COO-, NHCO-, NHCOO-, NHCONH- und /oder Mischungen davon und a = 1-20,
als Mittel zur Rheologiesteuerung.

7. Verwendung von Biuretverbindungen nach Anspruch 6 zur Thixotropierung von Beschichtungssystemen, als Antiablaufmittel und/oder Antiabsetzmittel.

## Claims

1. Biuret compounds of the general formula 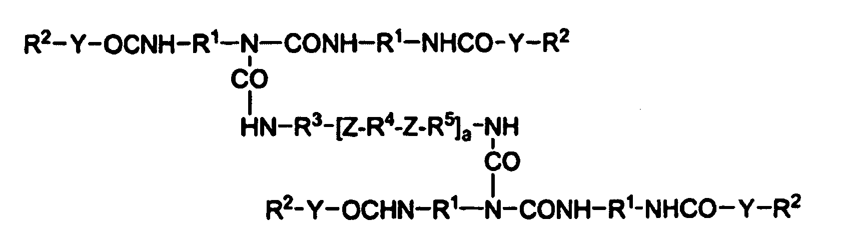 in which:
R¹ = C₂-C₁₈ alkylene, cycloalkylene, arylene or aralkylene,
Y = -O- and/or -NH-,
R² = C₄-C₂₂ alkyl, C₃-C₁₈ alkenyl, cycloalkyl, aralkyl, CₘH₂ₘ₊₁(O-CₙH₂ₙ)ₓ -(O-CH(C₆H₅)-CH₂)ᵤ-, CₘH₂ₘ₊₁(OOC-CᵥH₂ᵥ)ₓ-, X-C₆H₄-(O-CₙH₂ₙ)ₓ-(O-CH(C₆H₅)-CH₂)ᵤ-, wherein m = 1 - 22, n = 2 - 4, x = 0 - 15, u = 0 - 15, v = 4 - 5,
X = C₁-C₁₂ alkyl, -(C₆H₅)₁₋₄ and
R³, R⁴ and R⁵ = C₂-C₁₈ alkylene, cycloalkylene, arylene or aralkylene, wherein R³, R⁴ and R⁵ can be the same or different,
Z = -COO-, NHCO-, NHCOO-, NHCONH- and/or mixtures thereof,
and wherein a = 1 - 20.

2. Biuret compounds according to claim 1, **characterized in that** the radical R² is a polyalkoxymonoalcohol radical containing ethylene oxide and/or propylene oxide and/or butylene oxide groups.

3. Process for the preparation of the biuret compounds according to claim 1, **characterized in that** uretdiones of the general formula (A) 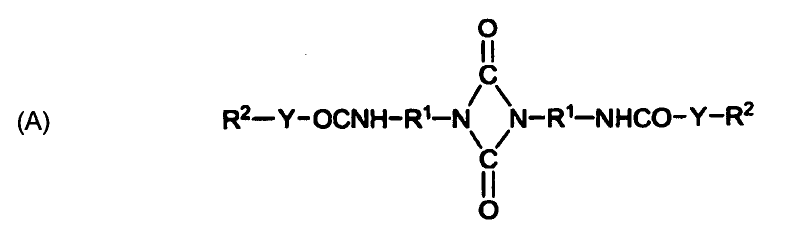 in which:
R¹ = C₂-C₁₈ alkylene, cycloalkylene, arylene or aralkylene,
Y = -O- and/or -NH-,
R² = C₄-C₂₂ alkyl, C₃-C₁₈ alkenyl, cycloalkyl, aralkyl, CₘH₂ₘ₊₁(O-CₙH₂ₙ)ₓ -(O-CH(C₆H₅)-CH₂)ᵤ-, CₘH₂ₘ₊₁(OOC-CᵥH₂ᵥ)ₓ-, X-C₆H₄-(O-CₙH₂ₙ)ₓ-(O-CH(C₆H₅)-CH₂)ᵤ-,
wherein m = 1 - 22, n = 2 - 4, x = 0 - 15, u = 0 - 15, v = 4 - 5, and X = C₁-C₁₂ alkyl or -(C₆H₅)₁₋₄,
are reacted with diamines of the general formula B
(B) H₂N-R³-[Z-R⁴-Z-R⁵]ₐ-NH₂
in which:
R³, R⁴ and R⁵ = C₂-C₁₈ alkylene, cycloalkylene, arylene or aralkylene,
wherein R³, R⁴ and R⁵ can be the same or different,
Z = -COO-, NHCO-, NHCOO-, NHCONH- and/or mixtures thereof,
and wherein a = 1 - 20.

4. Process for the preparation of the biuret compounds according to claim 3, **characterized in that** compounds of the formulae (A) and (B) are reacted at a reaction temperature between 60 °C and 120 °C.

5. Process for the preparation of the biuret compounds according to claims 3 and 4, **characterized in that** the molar ratio of the urethane and/or urea containing polymer to polyamine is between 1 : 0,3 and 1 : 0,7.

6. Use of biuret compounds of the general formula 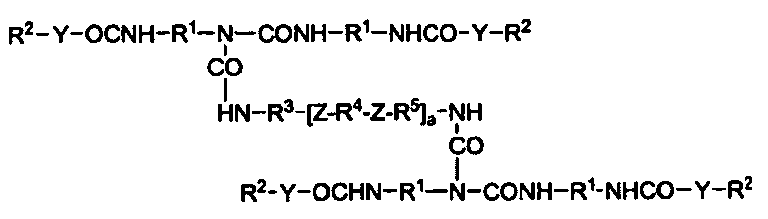 in which:
R¹ = C₂-C₁₈ alkylene, cycloalkylene, arylene or aralkylene,
Y = -O- and/or -NH-,
R² = C₄-C₂₂ alkyl, C₃-C₁₈ alkenyl, cycloalkyl, aralkyl, CₘH₂ₘ₊₁(O-CₙH₂ₙ)ₓ -(O-CH(C₆H₅)-CH₂)ᵤ-, CₘH₂ₘ₊₁(OOC-CᵥH₂ᵥ)ₓ-, X-C₆H₄-(O-CₙH₂ₙ)ₓ-(O-CH(C₆H₅)-CH₂)ᵤ-,
wherein m = 1 - 22, n = 2 - 4, x = 0 - 15, u = 0 - 15, v = 4 - 5,
X = C₁-C₁₂ alkyl, -(C₆H₅)₁₋₄ and
R³, R⁴ and R⁵ = C₂-C₁₈ alkylene, cycloalkylene, arylene or aralkylene,
wherein R³, R⁴ and R⁵ can be the same or different,
Z = -COO-, NHCO-, NHCOO-, NHCONH- and/or mixtures thereof and a = 1 - 20,
as reology control agents.

7. Use of biuret compounds according to claim 6 for thixotropizing coating systems, as anti-sag agents or anti-settling agents.

## Revendications

1. Composés de biuret de formule générale 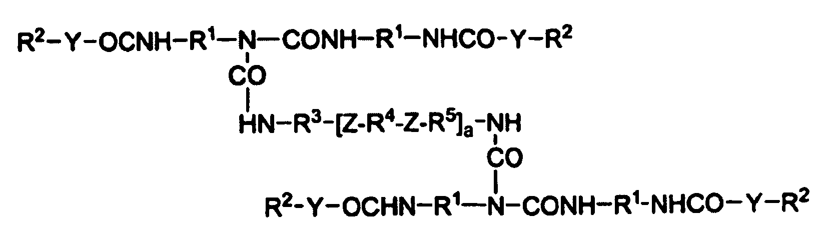 où:
R¹ = alkylène en C₂-C₁₈, cycloalkylène, arylène ou aralkylène,
Y = -O- et/ou -NH-,
R² = alkyle en C₄-C₂₂, alcényle en C₃-C₁₈, cycloalkyle, aralkyle, CₘH₂ₘ₊₁(O-CₙH₂ₙ)ₓ - (O-CH(C₆H₅)-CH₂)ᵤ-, CₘH₂ₘ₊₁(OOC-CᵥH₂ᵥ)ₓ-, X-C₆H₄-(O-CₙH₂ₙ)ₓ-(O-CH(C₆H₅)-CH₂)ᵤ-, où m = 1 1 à 22, n = 2 à 4, x = 0 à 15, u = 0 à 15, v = 4 à 5,
X = alkyle en C₁-C₁₂, -(C₆H₅)₁₋₄ et
R³, R⁴ et R⁵ = alkylène en C₂-C₁₈, cycloalkylène, arylène ou aralkylène, où R³, R⁴ et R⁵ peuvent être identiques ou différents,
Z = -COO-, NHCO-, NHCOO-, NHCONH- et/ou des mélanges de ceux-ci
et où a = 1 1 à 20.

2. Composés de biuret selon la revendication 1, **caractérisés en ce que** le groupe fonctionnel R² est un groupe fonctionnel monoalcool polyalcoxylique qui contient des groupements oxyde d'éthylène et/ou oxyde de propylène et/ou oxyde de butylène.

3. Procédé de fabrication des composés de biuret selon la revendication 1, **caractérisé en ce que** des urétdiones de formule générale (A) 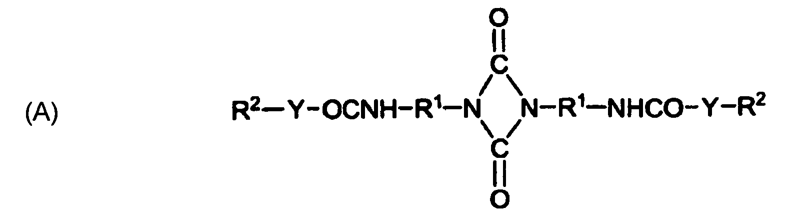 où:
R¹ = alkylène en C₂-C₁₈, cycloalkylène, arylène ou aralkylène,
Y = -O- et/ou -NH-,
R² = alkyle en C₄-C₂₂, alcényle en C₃-C₁₈, cycloalkyle, aralkyle, CₘH₂ₘ₊₁(O-CₙH₂ₙ)ₓ - (O-CH(C₆H₅)-CH₂)ᵤ-, CₘH₂ₘ₊₁(OCCᵥC₂ᵥ)ₓ-, X-C₆H₄-(O-CₙH₂ₙ)ₓ-(O-CH(C₆H₅)-CH₂)ᵤ-, où m = 1 à 22, n = 2 à 4, x = 0 à 15, u = 0 à 15, v = 4 à 5,
X = alkyle en C₁-C₁₂, -(C₆H₅)₁₋₄,
sont réagies avec des diamines de formule générale B
(B) H₂N-R³-[Z-R⁴-Z-R⁵]ₐ-NH₂
où :
R³, R⁴ et R⁵ = alkylène en C₂-C₁₈, cycloalkylène, arylène ou aralkylène, où R³, R⁴ et R⁵ peuvent être identiques ou différents,
Z = -COO-, NHCO-, NHCOO-, NHCONH- et/ou des mélanges de ceux-ci
et où a = 1 à 20.

4. Procédé de fabrication des composés de biuret selon la revendication 3, **caractérisé en ce que** des composés de formules (A) et (B) sont réagis à une température de réaction comprise entre 60 °C et 120 °C.

5. Procédé de fabrication des composés de biuret selon les revendications 3 et 4, **caractérisé en ce que** le rapport molaire du polymère contenant de l'uréthane et/ou de l'urée à la polyamine est compris entre 1 : 0,3 et 1 : 0,7.

6. Utilisation de composés de biuret de formule générale 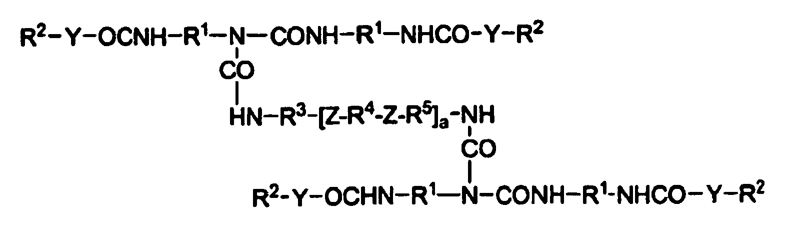 où:
R¹ = alkylène en C₂-C₁₈, cycloalkylène, arylène ou aralkylène,
Y = -O- et/ou -NH-,
R² = alkyle en C₄-C₂₂, alcényle en C₃-C₁₈, cycloalkyle, aralkyle, CₘH₂ₘ₊₁(O-CₙH₂ₙ)ₓ-(O-CH(C₆H₅)-CH₂)ᵤ-, CₘH₂ₘ₊₁(OOC-CᵥH₂ᵥ)ₓ-, X-C₆H₄-(O-CₙH₂ₙ)ₓ-(O-CH(C₆H₅)-CH₂)ᵤ-, où m = 1 à 22, n = 2 à 4 , x = 0 à 15, u = 0 à 15, v = 4 à 5,
X = alkyle en C₁-C₁₂, -(C₆H₅)₁₋₄ et
R³, R⁴ et R⁵ = alkylène en C₂-C₁₈, cycloalkylène, arylène ou aralkylène,
où R³, R⁴ et R⁵ peuvent être identiques ou différents,
Z = -COO-, NHCO-, NHCOO-, NHCONH- et/ou des mélanges de ceux-ci et a = 1 à 20,
en tant qu'agents de contrôle de la rhéologie.

7. Utilisation de composés de biuret selon la revendication 6 pour la thixotropisation de systèmes de revêtement, en tant qu'agents anti-écoulement et/ou agents de suspension.
